# EUROPEAN PATENT SPECIFICATION

(11) **EP 1 558 240 B1**
(45) Date of publication and mention of the grant of the patent: **31.05.2006**
(21) Application number: 03777932.9
(22) Date of filing: 28.10.2003
(51) Int. Cl.: A61K 31/336, A61P 3/10, C07D 493/08

(54) **HYPOESTOXIDES, DERIVATIVES AND AGONISTS THEREOF FOR USE IN THE TREATMENT AND PROPHYLAXIS OF HYPERLIPIDEMIA**
HYPOESTOXIDE, DERIVATE UND AGONISTEN DAVON ZUR VERWENDUNG BEI DER BEHANDLUNG UND PROPHYLAXE VON HYPERLIPIDÄMIE
HYPOESTOXYDES, DERIVES ET AGONISTES DE CES DERNIERS A UTILISER DANS LE TRAITEMENT ET LA PROPHYLAXIE D'HYPERLIPIDEMIES

(30) Priority: 28.10.2002 US 421533 P
(43) Date of publication of application: 03.08.2005
(73) Proprietor: Paraquest, Inc., Bloomington, CA 92316-0998 (US)
(72) Inventor: NCHEKWUBE, Emeka, J., Morgan Hill, CA 95037 (US); OJO-AMAIZE, Emmanuel, A., Glendora, CA 91740 (US); COTTAM, Howard, B., Escondido, CA 92027 (US)
(74) Representative: Banford, Paul Clifford
(86) International application number: PCT/US2003/034157
(87) International publication number: WO 2004/039362

(56) References cited:
- WO-A-01/01975
- US-A- 5 801 193
- E. A. OJO-AMAIZE ET AL: "hypoestoxide, a Natural Nonmutagenic Diterpenoid with Antiangiogenic and Antitumor Activity: Possible Mechanisms of Action" CANCER RESEARCH, vol. 62, 15 July 2002 (2002-07-15), pages 4007-4014, XP002279020

## Description

### FIELD OF THE INVENTION

This invention relates to the use of hypoestoxides, derivatives thereof for the manufacture of a medicament for the treatment and prophylaxis of hyperlipidemias, including hypercholesterolemia and hypertriglyceridemia.

### BACKGROUND OF THE INVENTION

Hyperlipidemias are conditions of abnormal plasma lipids, lipoproteins, and/or cholesterol levels, and include hypercholesterolemia and hypertriglyceridemia. Hyperlipidemias commonly accelerate atherosclerosis and predispose individuals to coronary heart disease. Hyperlipidemias can be inherited conditions or can be the result of a lifestyle that includes dietary excess, increased body weight and little or no vigorous exercise. (Jay H. Stein et al., Eds., Internal Medicine, 5^{th} Ed., 1998, p. 1892.) References of interest providing background information on hyperlipidemia include: Foxton et al., *Hyperlipidemia,* Nursing Standard (June 13, 1998) 12:49-56 and Krauss, *Triglycerides and Atherogenic Lipoproteins: Rationale for Lipid Management,* The American Journal of Medicine (July 6, 1998) 105:58S-62S. All publications, patents, and patent documents referenced herein are incorporated by reference in their entirety as if fully set forth.

The plasma lipids cholesterol and triglycerides are insoluble in aqueous solutions and thus cannot circulate freely in plasma. Instead they are complexed with specialized proteins called apolipoproteins, or apoproteins. Lipid-apoprotein complexes are named lipoproteins and are produced by the gut and liver but are extensively modified in the plasma. The major function of lipoproteins is to transport lipids.

Hypertriglyceridemia (HTG) is a common inherited disorder of lipid metabolism in humans that is characterized by a proatherogenic lipoprotein profile, including increased plasma triglycerides and very low density lipoproteins (VLDL), and often decreased high density lipoproteins (HDL). Whereas its frequency in the general population is about 1% (1), HTG occurs in about 5% of patients surviving a myocardial infarction, indicating an increased risk for atherosclerosis. HTG can also be the result of dietary factors (excessive intake of total calories, carbohydrates and alcohol), diseases (e.g., diabetes mellitus and chronic renal failure) and drugs (e.g., oral contraceptives and beta-blockers). (Jay H. Stein et al., Eds., Internal Medicine, 5^{th} Ed., 1998, pp. 1894-1895.)

Moreover, a major component of atherosclerotic plaques is cholesterol; this cholesterol is believed to be derived largely from plasma cholesterol. Hypercholesterolemia is defined as a high plasma cholesterol level. The profile of individuals with hypercholesterolemia includes an elevated total cholesterol level and an elevated level of low-density lipoproteins (LDL). Individuals with severe hypercholesterolemia (total cholesterol over 300 mg/dl and LDL over 220 mg/dl) have a risk of coronary heart disease that is at least four times the baseline risk in the general population. (Jay H. Stein et al., Eds., Internal Medicine, 5^{th} Ed., 1998, pp. 1892-1894.)

Further, hyperlipidemia is an important complication after organ transplantation and contributes to the development of post-transplant accelerated coronary artery diseases. Successful organ transplantation in humans requires the administration of pharmacologic immunosuppressants for prophylaxis of acute organ rejection. Cyclosporin A (CsA) and tacrolimus are now routinely used for transplantation of all solid organ and bone marrow transplantation. However, these agents are important causes of post-transplant hypertension, hyperlipidemia, and diabetes, all of which contribute to morbidity and mortality in the transplant recipient. (See, e.g., Akhlaghi, et al., *Risk Factors for the Development and Progression of Dyslipidemia After Heart Transplantation,* Transplantation (2002) 73:1258-1264.)

Because of their link with vascular disease, a number of approaches for controlling hyperlipidemias have been developed. Such approaches include changes in lifestyle, such as improved diet and increased exercise, as well as drug therapy. Drugs finding use in the management of plasma lipid profiles include: bile acid binding resins, niacin, HMG-CoA reductase inhibitors (statins) and fibric acid derivatives (e.g., gemfibrozil).

Despite the development of the above approaches, there continues to be a need for the identification of new treatment therapies for hyperlipidemias.

Examples of the use of diterpene compounds (e.g., hypoestoxides) for the treatment and prophylaxis of various conditions include U.S. Patent No. 5,801,193 (inflammation, graft rejection, graft-versus-host disease and T-cell mediated autoimmune disorders), U.S. Patent No. 6,242,484 (anti-parasitic therapy and prophylaxis), U.S. Patent No. 5,994,328 (inhibiting tumor growth) and U.S. Patent No. 6,001,871 (antiviral therapy).

### SUMMARY OF THE INVENTION

It is previously undisclosed in the art to use hypoestoxides and other diterpene compounds for the treatment and prophylaxis of hyperlipidemias. Thus, the present invention provides the use of hypoestoxides and derivatives thereof for the manufacture of a medicament for treating a host, such as a human, suffering from hyperlipidemia resulting from elevated plasma levels of cholesterol, triglycerides and/or lipoproteins such as VLDL, such that those pathological condition(s) are ameliorated thereby. In addition, the use of hypoestoxides and derivatives thereof for the manufacture of a medicament for prophylaxis against the development of hyperlipidemias commonly observed in recipients of solid organ or bone marrow transplants are provided. Thus, the use comprises administering to the afflicted host or transplant recipient a therapeutically, or prophylactically, effective amount of a compound having the formula I: 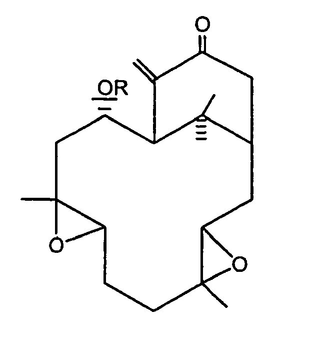 or pharmaceutically acceptable salts thereof, wherein R is:
a) H or acetyl,
b) P(O)(OH)₂,
c) P(O)(OH)(OM), wherein M is selected from the group consisting of an alkali metal salt and an alkaline earth metal salt,
d) P(O)OM₂ wherein M is each independently selected from the group consisting of alkali metal salts and alkaline earth metal salts,
e) Alkyl of 1 to 12 carbon atoms having 0 to 6 double bonds, said alkyl selected from the group consisting of substituted, unsubstituted, straight chain and branched alkyls,
f) (CH₂)n morpholine, wherein n=1-4,
g) morpholinomethylphenyl, ortho-aminophenyl or ortho-hydroxyphenyl,
h) (CH₂)n COOR₂ wherein n=1-4, R₂ is each selected from the group consisting of H, an alkali metal salt, an alkaline earth metal salt, NH₄ + and N+(R₃)₄ wherein R₃ is each independently selected from the group consisting of H and an alkyl of 1 to 4 carbon atoms, or
i) COR₁ wherein R₁ is selected from the group consisting of H, (CH₂)n CH₃ wherein n=0-6, (CH₂)n COOR₂ wherein n=1-4 and R₂ is each selected from the group consisting of H, an alkali metal salt, an alkaline earth metal salt, NH₄ + and N+(R₃)₄, and (CH₂)n N+(R₃)₄, wherein n=1-4 and R₃ is each independently selected from the group consisting of H and an alkyl of 1 to 4 carbon atoms,
and wherein the effective amount is an amount sufficient to ameliorate at least one symptom of said disease, and compounds may be used alone or in combination with other chemotherapeutic agents.

### DETAILED DESCRIPTION OF THE INVENTION

Uses of treating a host suffering from hyperlipidemia are provided. In the subject uses, an effective amount of an agent as described above is administered to an afflicted host, in an amount sufficient to ameliorate at least one condition related to or included under the definition of hyperlipidemias. As used herein, the term "host" or "subject" is taken to mean human, as well as other animals. The term "ameliorate" means to improve, lessen the severity of or mitigate. Also provided are uses for prophylactically treating a host patient at risk of a hyperlipidemic condition, such as a transplant patient, with an agent as described above, in combination with standard chemotherapeutic agents, such as CyA or tacrolimus. The uses comprise administering to the afflicted host or host patient a therapeutically, or prophylactically, effective amount of a compound having the formula I: 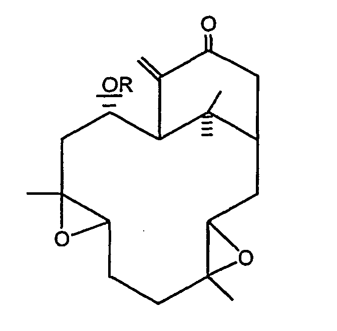 or pharmaceutically acceptable salts thereof, wherein R is:
a) H or acetyl,
b) P(O)(OH)₂,
c) P(O)(OH)(OM), wherein M is selected from the group consisting of an alkali metal salt and an alkaline earth metal salt,
d) P(O)OM₂ wherein M is each independently selected from the group consisting of alkali metal salts and alkaline earth metal salts,
e) Alkyl of 1 to 12 carbon atoms having 0 to 6 double bonds, said alkyl selected from the group consisting of substituted, unsubstituted, straight chain and branched alkyls,
f) (CH₂)n morpholine, wherein n=1-4,
g) morpholinomethylphenyl, ortho-aminophenyl or ortho-hydroxyphenyl,
h) (CH₂)n COOR₂ wherein n=1-4, R₂ is each selected from the group consisting of H, an alkali metal salt, an alkaline earth metal salt, NH₄ + and N+(R₃)₄ wherein R₃ is each independently selected from the group consisting of H and an alkyl of 1 to 4 carbon atoms, or
i) COR₁ wherein R₁ is selected from the group consisting of H, (CH₂)n CH₃ wherein n=0-6, (CH₂)n COOR₂ wherein n=1-4 and R₂ is each selected from the group consisting of H, an alkali metal salt, an alkaline earth metal salt, NH₄ + and N+(R₃)₄, and (CH₂)n N+(R₃)₄, wherein n=1-4 and R₃ is each independently selected from the group consisting of H and an alkyl of 1 to 4 carbon atoms,
and wherein the effective amount is an amount sufficient to ameliorate at least one symptom of said disease, and compounds may be used alone or in combination with other chemotherapeutic agents.

Preferred embodiments of the invention are compounds of formula I, wherein R = H or R = acetyl (hypoestoxide).

The magnitude of a prophylactic or therapeutic dose of compounds of formula I in the treatment or prevention of hyperlipidemia may vary with the progression of the disease, the chemotherapeutic agent(s) or other therapy used, and the route of administration. The dose, and perhaps the dose frequency, may also vary according to the age, body weight, and response of the individual patient. In general, the total daily dose range for compounds of formula I, for the conditions described herein, may be from about 0.5 mg to about 5000 mg, in single or divided doses. Preferably, a daily dose range may be about 1 mg to about 4000 mg, in single or divided doses. In managing the patient, the therapy may be initiated at a lower dose and may be subsequently increased depending on the patient's global response. Patients, including but not limited to, infants, children, patients over 65 years, and those with impaired renal or hepatic function may initially receive lower doses. Doses for these patients may be titrated based on global response and blood level. It is possible to use dosages outside these ranges in some cases. Further, it is noted that it will be readily apparent to the clinician or treating physician how and when to interrupt, adjust or terminate therapy in conjunction with individual patient response. The term "an effective amount" is meant to encompass the above-described dosage amounts and dose frequency schedule.

Any suitable route of administration may be employed for providing the patient with an effective amount of compounds of formula I. For example, and without limitation, oral, rectal, parenteral (subcutaneous, intravenous, intramuscular), intrathecal, transdermal, and similar forms of administration may be employed. Dosage forms may include tablets, troches, dispersions, suspensions, solutions, capsules and patches. The compound may be administered prior to, concurrently with, or after administration of other chemotherapy, or continuously (i.e., in daily doses, during all or part of, a chemotherapy regimen, such as a CsA regimen.) The compound, in some cases, may be combined with the same carrier or vehicle used to deliver the other chemotherapeutic agent.

Thus, the compounds of the present invention may be systemically administered (e.g., orally, in combination with a pharmaceutically acceptable vehicle such as an inert diluent or an assimilable edible carrier). The compounds of the present invention may be enclosed in hard or soft shell gelatin capsules, may be compressed into tablets, or may be incorporated directly with the food of the patient's diet. For oral therapeutic administration, the active compound may be combined with one or more excipients and used in the form of, inter alia, ingestible tablets, buccal tablets, troches, capsules, elixirs, suspensions, syrups or wafers. Such compositions and preparations may contain at least 0.1% of an active compound of the present invention. The percentage of the compositions and preparations may, of course, be varied and may conveniently be between about 2 to about 60% of the weight of a given unit dosage form. The amount of active compound in such therapeutically or prophylactically useful compositions is such that an effective dosage level will be obtained.

The tablets, troches, pills, capsules, and the like may also contain the following:
binders such as gum tragacanth, acacia, corn starch or gelatin, excipients such as dicalcium phosphate, disintegrating agents such as corn starch, potato starch and alginic acid, lubricants such as magnesium stearate, sweetening agents such as sucrose, fructose, lactose or aspartame, or a flavoring agent such as peppermint, oil of wintergreen, or cherry flavoring. When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with, inter alia, gelatin, wax, shellac or sugar. A syrup or elixir may contain the active compound, sucrose or fructose as a sweetening agent, methyl and
propylparabens as preservatives, a dye and flavoring such as cherry or orange flavor. Of course, any material used in preparing any unit dosage form should be pharmaceutically acceptable and substantially non-toxic in the amounts employed. In addition, the active compound may be incorporated into sustained-release preparations and devices.

The active compound may also be administered intravenously or intraperitoneally by infusion or injection. Solutions of the active compound or its salts can be prepared in water, optionally mixed with a non-toxic surfactant. Dispersions can also be prepared in glycerol, liquid polyethylene glycols, triacetin, and mixtures thereof and in oils. Under ordinary conditions of storage and use, these preparations may contain a preservative to prevent the growth of microorganisms.

The pharmaceutical dosage forms suitable for injection or infusion may include sterile aqueous solutions or dispersions or sterile powders comprising the active ingredient which are adapted for the extemporaneous preparation of sterile injectable or infusible solutions or dispersions, optionally encapsulated in liposomes. In all cases, the ultimate dosage form should be sterile, fluid and stable under the conditions of manufacture and storage. The liquid carrier or vehicle may be a solvent or liquid dispersion medium comprising, for example, water, ethanol, a polyol (for example, glycerol, propylene glycol, liquid polyethylene glycols, and the like), vegetable oils, non-toxic glyceryl esters, and suitable mixtures thereof. The proper fluidity can be maintained, such as, for example, by the formation of liposomes, by the maintenance of the required particle size in the case of dispersions or by the use of surfactants. The prevention of the action of microorganisms can be brought about by various antibacterial and antifungal agents (e.g., parabens, chlorobutanol, phenol, sorbic acid and thimerosal). In many cases, it may be preferable to include isotonic agents, for example, sugars; buffers or sodium chloride. Prolonged absorption of the injectable compositions can be brought about by the use in the compositions of agents delaying absorption, such as, for example, aluminum monostearate and gelatin.

Sterile injectable solutions of the compounds of the present invention may be prepared by incorporating the active compound in the required amount in an appropriate solvent with various other ingredients enumerated above, as required, followed by filter sterilization. In the case of sterile powders for the preparation of sterile injectable solutions, the preferred methods of preparation are vacuum drying and freeze drying techniques, which yield a powder of the active ingredient plus any additional desired ingredient present in the previously sterile-filtered solutions.

Useful dosages of the compounds of formula I may be determined by comparing their *in vivo* activity in animal models. Methods for the extrapolation of effective dosages in mice and other animals to humans are known in the art (see, e.g., U.S. Patent No. 4,938,949).

### EXAMPLES

In one example, hypoestoxide was administered orally to a 2-year-old female beagle dog at a dose of 30 mg/kg, once daily for seven days. Blood cholesterol and triglyceride levels were determined in sera obtained at days 1, 3, 7 and 14, respectively. Assays were performed by a standard colorimetric method by a commercial laboratory (Antech Diagnostics, Irvine, CA). The administration of hypoestoxide significantly lowered blood cholesterol and triglyceride levels, as shown in Table 1.

**Table 1: The effect of oral administration of hypoestoxide on blood cholesterol and triglyceride levels in dogs**

| **Days →** | **-1** | **1** | **3** | **7** | **14** | **Normal Reference Range** |
|---|---|---|---|---|---|---|
| **Cholesterol (mg/dl)** | 308 | 122 | 130 | 124 | 134 | (92-324) |
| **Triglycerides (mg/dl)** | 300 | 296 | 70 | 33 | 51 | (29-291) |

In another example, oral ingestion of a 1g capsule of dried leaf powder of hypoestes rosea shrub (parent plant of hypoestoxide), taken once daily for one year as a dietary supplement, significantly lowered blood cholesterol and triglyceride levels in a human subject, as shown in Table 2.

**Table 2: The effect of oral ingestion by humans of dried leaf powder of hypoestes rosea shrub, containing 0.1% hypoestoxide**

| **Year →** | **-1** | **1** | **Normal Reference Range** |
|---|---|---|---|
| **Cholesterol (mg/dl)** | 215 | 206 | (50-200) |
| **Triglycerides (mg/dl)** | 103 | 96 | (30-210) |

## Claims

1. Use of an effective amount of a compound having the formula: 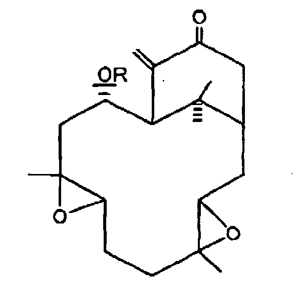 and pharmaceutically acceptable salts thereof, wherein R is selected from the group consisting of:
a) H or acetyl,
b) P(O)(OH)₂,
c) P(O)(OH)(OM), wherein M is selected from the group consisting of an alkali metal salt and an alkaline earth metal salt,
d) P(O)OM₂ wherein M is each independently selected from the group consisting of alkali metal salts and alkaline earth metal salts,
e) Alkyl of 1 to 12 carbon atoms having 0 to 6 double bonds, said alkyl selected from the group consisting of substituted, unsubstituted, straight chain and branched alkyls,
f) (CH₂)n morpholine, wherein n=1-4,
g) morpholinomethylphenyl, ortho-aminophenyl or ortho-hydroxyphenyl,
h) (CH₂)n COOR₂ wherein n=1-4, R₂ is each selected from the group consisting of H, an alkali metal salt, an alkaline earth metal salt, NH₄ + and N+(R₃)₄ wherein R₃ is each independently selected from the group consisting of H and an alkyl of 1 to 4 carbon atoms, and
i) COR₁ wherein R₁ is selected from the group consisting of H, (CH₂)n CH₃ wherein n=0-6, (CH₂)n COOR₂ wherein n=1-4 and R₂ is each selected from the group consisting of H, an alkali metal salt, an alkaline earth metal salt, NH₄+ and N+(R₃)₄, and (CH₂)n N+(R₃)₄, wherein n=1-4 and R₃ is each independently selected from the group consisting of H and an alkyl of 1 to 4 carbon atoms;
in the manufacture of a medicament for treating a host having hyperlipidemia or protecting a host from developing hyperlipidemia.

2. The use according to claim 1 wherein the compound is used in combination with other chemotherapeutic agents.

3. The use according to claim 1 wherein R is selected from the group consisting of H and acetyl.

4. The use according to claim 3 wherein the hyperlipidemia is selected from the group consisting of hypertriglyceridemia and hypercholesterolemia.

5. The use according to claim 1 wherein the daily dose range of the compound is from about 0.5 mg to about 5000 mg.

6. The use according to claim 1 further including incorporating the compound in a dosage form selected from the group consisting of a tablet, a troche, a dispersion, a suspension, a solution, a capsule, a patch, a syrup, an elixir and a wafer.

7. The use according to claim 6 wherein the dosage form contains at least 0.1% by weight of the compound.

8. The use according to claim 2 wherein the medicament is used to protect a host from developing hyperlipidemia and the other chemotherapeutic agents are selected from the group consisting of Cyclosporin A and tacrolimus.

9. The use according to claim 1 wherein the medicament is used to protect a host from developing hyperlipidemia and said host is at risk of developing hyperlipidemia due to recent solid organ or bone marrow transplantation.

## Patentansprüche

1. Verwendung einer wirksamen Menge einer Verbindung der Formel: 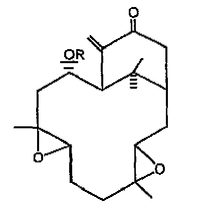 und pharmazeutisch akzeptabler Salze derselben, wobei R aus der Gruppe ausgewählt ist bestehend aus:
a) H oder Acetyl,
b) P(O)(OH)₂,
c) P(O)(OH)(OM), wobei M aus der Gruppe ausgewählt ist bestehend aus einem Alkalimetallsalz und einem Erdalkalimetallsalz,
d) P(O)OM₂, wobei jedes M jeweils unabhängig aus der Gruppe ausgewählt ist bestehend aus Alkalimetallsalzen und Erdalkalimetallsalzen.
e) Alkyl mit 1 bis 12 Kohlenstoffatomen, die 0 bis 6 Doppelbindungen aufweisen, wobei das Alkyl aus der Gruppe ausgewählt ist, bestehend aus substituierten, unsubstituierten, geradkettigen und verzweigten Alkylen,
f) (CH₂)n-Morpholin, wobei n = 1- 4 ist,
g) Mopholinmethylphenyl, Orthoaminophenyl oder Orthohydroxyphenyl,
h) (CH₂)n COOR₂, wobei n = 1 - 4, jedes R₂ aus der Gruppe ausgewählt ist, bestehend aus H, einem Alkalimetallsalz, einem Erdalkalimetallsalz, NH₄+ und N+(R₃)₄, wobei jedes R₃ unabhängig aus der Gruppe ausgewählt ist, bestehend aus H und einem Alkyl mit 1 bis 4 Kohlenstoffatomen und
i) COR₁, wobei R₁ aus der Gruppe ausgewählt ist, bestehend aus H, (CH₂)ₙCH₃, wobei n = 0 - 6, (CH₂)n COOR₂, wobei n = 1 - 4 und jedes R₂ aus der Gruppe ausgewählt ist, bestehend aus H, einem Alkalimetallsalz, einem Erdalkalimetallsalz, NH₄ + und N+(R₃)₄ und (CH₂)ₙN+(R₃)₄, wobei n = 1 - 4 und jedes R₃ unabhängig ausgewählt ist aus der Gruppe bestehend aus H und einem Alkyl mit 1 bis 4 Kohlenstoffatomen;
bei der Herstellung eines Medikaments zur Behandlung eines Wirts, der an Hyperlipidämie leidet oder zum Schutz eines Wirts gegen das Entwickeln von Hyperlipidämie.

2. Verwendung nach Anspruch 1, wobei die Verbindung in Kombination mit anderen chemotherapeutischen Mitteln verwendet wird.

3. Verwendung nach Anspruch 1, wobei R aus der Gruppe ausgewählt ist, bestehend aus H und Acetyl,

4. Verwendung nach Anspruch 3, wobei die Hyperlipidämie aus der Gruppe ausgewählt ist, bestehend aus Hypertriglyceridämie und Hypercholesterinämie.

5. Verwendung nach Anspruch 1, wobei der Tagesdosisbereich der Verbindung ca. 0,5 mg bis ca. 5000 mg beträgt.

6. Verwendung nach Anspruch 1, des Weiteren das Einarbeiten der Verbindung in eine Dosierungsform ausgewählt aus der Gruppe bestehend aus einer Tablette, einer Pastille, einer Dispersion, einer Suspension, einer Lösung, einer Kapsel, einem Pflaster, einem Sirup, einem Elixier und einer Waffel beinhaltend.

7. Verwendung nach Anspruch 6, wobei die Dosierungsform mindestens 0,1 Gew: % der Verbindung enthält.

8. Verwendung nach Anspruch 2, wobei das Medikament dazu verwendet wird, um einen Wirt gegen das Entwickeln von Hyperlipidämie zu schützen, und die anderen chemotherapeutischen Mittel aus der Gruppe ausgewählt sind bestehend aus Cyclosporin A und Takrolismus.

9. Verwendung nach Anspruch 1, wobei das Medikament zum Schutz eines Wirts gegen die Entwicklung von Hyperlipidämie verwendet wird und der Wirt der Gefahr ausgesetzt ist, Hyperlipidämie auf Grund einer kürzlichen Transplantation eines festen Organs oder von Knochenmark zu entwickeln.

## Revendications

1. Utilisation d'une quantité efficace d'un composé répondant à la formule : 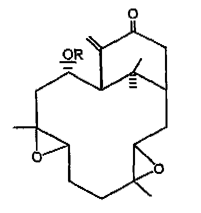 et de sels pharmaceutiquement acceptables de celui-ci, où R est sélectionné parmi le groupe consistant en :
a) H ou acétyle,
b) P(O)(OH)₂,
c) P(O)(OH)(OM), où M est sélectionné parmi le groupe consistant en un sel d'un métal alcalin et un sel d'un métal alcalino-terreux,
d) P(O)OM₂, où chaque M est indépendamment sélectionné parmi le groupe consistant en des sels d'un métal alcalin ou des sels d'un métal alcalino-terreux,
e) alkyle à 1 à 12 atomes de carbone qui comporte de 0 à 6 doubles liaisons, ledit groupement alkyle étant sélectionné parmi le groupe consistant en des alkyles substitués, non substitués, à chaîne linéaire et ramifiés,
f) (CH₂)ₙ-morpholine, où n = 1-4,
g) morpholinométhylphényle ortho-aminophényle ou ortho-hydroxyphényle,
h) (CH₂)ₙCOOR₂, où n = 1-4, chaque R₂ est sélectionné parmi le groupe consistant en H, un sel d'un métal alcalin, un sel d'un métal alcalino-terreux, NH₄⁺ et N⁺(R₃)ₙ, où chaque R₃ est indépendamment sélectionné parmi le groupe consistant en H et un alkyle à 1 à 4 atomes de carbone, et
i) COR₁, où R₁ est sélectionné parmi le groupe consistant en H, (CH₂)ₙCH₃ où n=0-6, (CH₂)ₙCOOR₂, où n = 1-4 et chaque R₂ est sélectionné parmi le groupe consistant en H, un sel d'un métal alcalin, un sel d'un métal alcalino-terreux, NH₄⁺ et N⁺(R₃)₄, et (CH₂)ₙN⁺(R₃)₄ où n = 1-4 et chaque R₃ est indépendamment sélectionné parmi le groupe consistant en H et un alkyle à 1 à 4 atomes de carbone ;
dans la fabrication d'un médicament indiqué dans le traitement d'un hôte atteint d'une hyperlipidémie ou dans la protection d'un hôte contre le développement d'une hyperlipidémie.

2. Utilisation selon la revendication 1, où le composé est employé en combinaison avec d'autres agents chimiothérapeutiques.

3. Utilisation selon la revendication 1, où R est sélectionné parmi le groupe consistant en H et un groupement acétyle.

4. Utilisation selon la revendication 3, où l'hyperlipidémie est sélectionnée parmi le groupe consistant en une hypertriglycéridémie et une hypercholestérolémie.

5. Utilisation selon la revendication 1, où l'éventail des doses quotidiennes du composé est compris entre environ 0,5 mg et environ 5 000 mg.

6. Utilisation selon la revendication 1, qui comporte également l'incorporation du composé dans une forme de dosage sélectionnée parmi le groupe consistant en un comprimé, une pastille, une dispersion, une suspension, une solution, une capsule, un pansement, un sirop, un élixir et un cachet ultra-fondant.

7. Utilisation selon la revendication 6, où la forme de dosage contient le composé à 0,1 % en poids au moins.

8. Utilisation selon la revendication 2, où le médicament est employé pour protéger un hôte contre le développement d'une hyperlipidémie et les autres agents chimiothérapeutiques sont sélectionnés parmi le groupe consistant en la ciclosporine A et le tacrolimus.

9. Utilisation selon la revendication 1, où le médicament est employé pour protéger un hôte contre le développement d'une hyperlipidémie et ledit hôte est à risque de développer une hyperlipidémie, du fait d'une transplantation récente d'un organe solide ou d'une greffe de moelle osseuse.
